# EUROPEAN PATENT APPLICATION

(11) **EP 4 736 771 A1**
(43) Date of publication of application: **06.05.2026**
(21) Application number: 24831475.9
(22) Date of filing: 20.05.2024
(51) Int. Cl.: A61B 6/03

(54) **VIBRATION MEASUREMENT DEVICE FOR X-RAY COMPUTED TOMOGRAPHY DIAGNOSTIC DEVICE**

(30) Priority: 29.06.2023 JP 2023107031
(71) Applicant: Canon Electron Tubes & Devices Co., Ltd., Otawara-shi, Tochigi 324-8550 (JP)
(72) Inventor: WATANABE Hazuo, Otawara-shi, Tochigi 324-8550 (JP); MURAKOSHI Yuuichi, Otawara-shi, Tochigi 324-8550 (JP)
(74) Representative: Hancox, Jonathan Christopher
(86) International application number: PCT/JP2024/018505
(87) International publication number: WO 2025/004603

(57) **Abstract**

To provide a vibration measuring device of an X-ray computed tomography apparatus allowing for improved quality.

A vibration measuring device (30) of an X-ray computed tomography apparatus, which includes a casing, a platform (13) configured to rotate in the casing, and an X-ray tube (15) arranged on the platform (13), is provided. A detector (33) configured to detect vibration in a state of the platform (13) rotating is arranged on at least one of the platform (13) and the X-ray tube (15).

## Description

### Technical Field

The embodiment according to the present invention relates to a vibration measuring device of an X-ray computed tomography apparatus.

### Background Art

A general structural body has a natural resonant frequency. Due to the resonance phenomenon occurring within the resonant frequency range, large vibration and noise may be generated, leading to the damage of the structural body itself in some cases.

In a computed tomography apparatus (hereinafter referred to as X-ray CT (computed tomography) apparatus), an X-ray tube and a cooling system, which have mass, are installed on a platform called gantry. The X-ray CT apparatus performs cross-sectional imaging of a test object by emitting X-rays while rotating the platform in a casing.

In the X-ray CT apparatus, vibration is generated because the X-ray tube has an anode rotation mechanism part, the cooling system has a circulation pump for coolant circulation, and the platform itself rotates.

In the X-ray CT apparatus in the prior art, since the expected output of the X-ray tube is small, the X-ray tube is compact, has low rigidity, and has a low mass. Therefore, the change in the vibration values caused by the operation of the X-ray tube has little impact on the X-ray CT apparatus as a whole. In recent years, with the higher performance of the X-ray CT apparatus, the expected output of the X-ray tube has been higher, and in response, the X-ray tube has increased in size, rigidity, and mass. Moreover, the platform has increased in rotation speed, and the change in the vibration values caused by the operation of the X-ray tube has increased.

In general, the vibration frequency of a vibration source does not change, without deformation of the structural material or change in the rigidity of the vibration source.

However, ninety-nine percent of the electric energy supplied to the X-ray tube for generating X-rays is converted into heat, and the heat causes deformation of the material and change in the rigidity of the anode part of the X-ray tube, resulting in change in resonant frequency.

In the case where the change in resonant frequency leads to change in the vibration frequency generated by the vibration source, and thus interference with a resonant frequency of another structure occurs, resonance phenomena may occur, thereby leading to abnormal noise, the fluctuation in tube current caused by the vibration of the filament of the X-ray tube, damage over time, and the like.

### Citation List

### Patent Literature

PTL 1: Japanese Laid-open Patent Publication No. 2020-13715

### Summary of Invention

### Technical Problem

The problem to be solved by the present invention is to provide a vibration measuring device of an X-ray computed tomography apparatus allowing for improved quality.

### Solution to Problem

The present embodiment is a vibration measuring device of an X-ray computed tomography apparatus including a casing, a platform configured to rotate in the casing, and an X-ray tube installed on the platform, a detector, arranged on either one or both of the platform and the X-ray tube, and configured to detect vibration in a state of the platform rotating.

### Brief Description of Drawings

[Fig. 1] Fig. 1 is a perspective view of an X-ray computed tomography apparatus in one embodiment.
[Fig. 2] Fig. 2 is a cross-sectional view of a rotating part of the X-ray computed tomography apparatus, viewed from the front direction.
[Fig. 3] Fig. 3 is a cross-sectional view of the rotating part of the X-ray computed tomography apparatus, viewed from the lateral direction.
[Fig. 4] Fig. 4 is a block diagram of a vibration measuring device of the X-ray computed tomography apparatus.
[Fig. 5] Fig. 5 shows examples of graphical displays of the measurement results obtained by the vibration measuring device: Fig. 5(A) is an explanatory diagram graphically displaying the chronological transition of the detection data synchronized with the rotation of a platform in the first state in which the X-ray tube is not emitting X-rays; and Fig. 5 (B) is an explanatory diagram graphically displaying the chronological transition of the detection data synchronized with the rotation of the platform in the second state in which the X-ray tube is emitting X-rays.
[Fig. 6] Fig. 6 is a waveform diagram of the measurement result obtained by the vibration measuring device.

### Description of Embodiment

Hereinafter, one embodiment will be described by referring to the drawings.

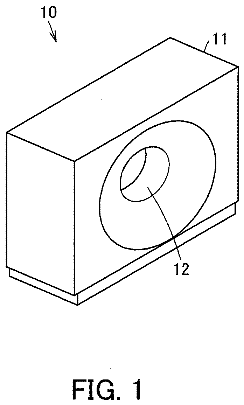

Fig. 1 shows a perspective view of an X-ray computed tomography apparatus 10 (hereinafter, referred to as x-ray CT apparatus 10).

The X-ray CT apparatus 10 includes a casing 11 serving as the outer enclosure of the X-ray CT apparatus 10. At the center of the casing 11, there is an introduction port 12 for introducing a test object. The X-ray CT apparatus 10 further includes a bed not illustrated, which allows a test object to be placed on it and facilitates introduction into the introduction port 12.

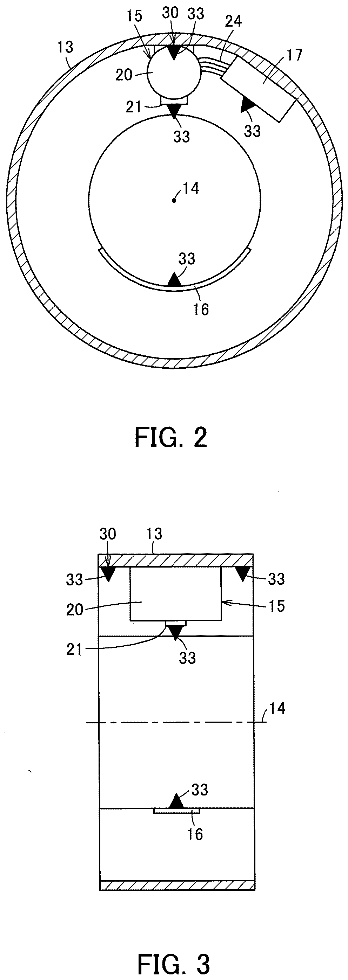

Fig. 2 shows a cross-sectional view of a rotating part of the X-ray CT apparatus 10, viewed from the front direction, while Fig. 3 shows a cross-sectional view of the rotating part of the X-ray CT apparatus 10, viewed from the lateral direction.

The casing 11 houses a platform (rotating platform) 13, which is the rotating part called gantry and is arranged rotatably around a virtual rotation axis 14. The rotation axis 14 coincides with the center axis of the introduction port 12 of the casing 11. The platform 13 has a ring shape, androtates around the rotation axis 14 by means of a rotational drive mechanism having a motor not illustrated as a drive part. The platform 13 rotates at high speed, for example, one rotation every 0.5 seconds.

On the inner wall of the outer peripheral frame of the platform 13, there are an X-ray tube 15, an X-ray detector 16, a cooling system 17, and the like. The X-ray tube 15, the X-ray detector 16, the cooling system 17, and the like rotate integrally with the platform 13.

The X-ray tube 15 is attached in a central area in the axial direction along the rotation axis 14 of the platform 13, on the inner wall of the outer peripheral frame of the platform 13. The X-ray tube 15 is a rotating anode X-ray tube. The X-ray tube 15 includes a cathode which emits electrons, an anode target which generates X-rays by the collision of the electrons emitted by the cathode, a rotating body which rotates while supporting the anode target, a coolant passage which is provided on the inner side of the rotating body, a vacuum vessel 20 which houses them, an X-ray transmission window 21 which is provided on the vacuum vessel 20 and emitsthe X-rays generated by the anode target to the outside, a coil which generates a magnetic field for rotating the rotating body from the outside of the vacuum vessel 20, and the like. The platform 13 may be equipped with a high voltage generating power source which applies a high voltage between the cathode and the anode target.

The X-ray tube 15 is arranged so that the axial direction of the rotation axis of the rotating body coincides with the axial direction of the rotation axis 14 of the platform 13, and further so that the X-ray transmission window 21 faces the rotation axis 14 serving as the rotation center of the platform 13. In the front view of the platform 13 (when viewed from the front direction of the X-ray CT apparatus 10 in Fig. 1), the cathode, the anode target, and the like of the X-ray tube 15 are arranged on the front side (on the near side), and the rotating body, the coil, and the like of the X-ray tube 15 are arranged on the rear side (on the far side).

The X-ray detector 16 is arranged at the position facing the X-ray transmission window 21 of the X-ray tube 15 on the platform 13, via the rotation axis 14 serving as the rotation center of the platform 13. The X-ray detector 16 detects the X-rays which have been emitted by the X-ray tube 15 and have penetrated a test object, and converts the detected X-rays into electrical signals and outputs them. The platform 13 may be equipped with a power source part which operates the X-ray detector 16, and a data collection device which amplifies the electrical signals output from the X-ray detector 16 and performs A/D conversion.

The cooling system 17 is arranged on the side of the X-ray tube 15 on the inner wall of the outer peripheral frame of the platform 13. The cooling system 17 is connected to the coolant passage of the X-ray tube 15 through a connection tube 24, and forms a circulation path for circulating coolant to and from the X-ray tube 15. The cooling system 17 includes a heat exchanger which dissipates heat from the coolant circulated through the circulation path to cool the coolant, and a circulation pump which circulates the coolant in the circulation path. The platform 13 may be equipped with a power source part which drives a fan of the heat exchanger and the circulation pump. The X-ray tube 15 and the cooling system 17 may be provided as one unit.

There is a slip ring not illustrated, which is provided between the platform 13 and the casing 11 which rotatably supports the platform 13, and the slip ring is configured to supply power and control signals from the outside to the devices installed on the platform 13, and to extract signals from the devices installed on the platform 13 to the outside.

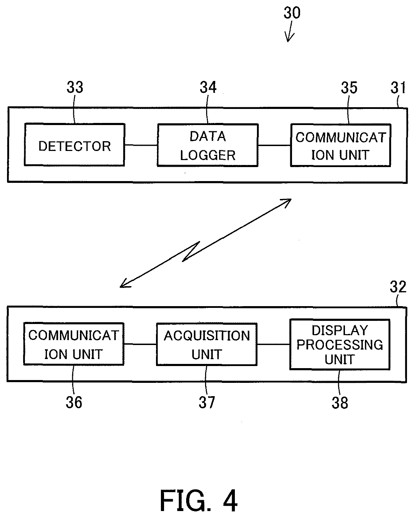

Fig. 4 shows a block diagram of a vibration measuring device 30 of the X-ray CT apparatus 10.

The vibration measuring device 30 includes an internal device 31 arranged on the platform 13, and an external device 32 arranged outside the X-ray CT apparatus 10.

The internal device 31 includes a plurality of detectors 33, a data logger 34, and a communication unit (internal communication unit) 35. The internal device 31 may operate by using the power supplied to the side of the platform 13 via the slip ring. Alternatively, the internal device 31 may operate on power from a battery arranged on the platform 13.

As the detectors 33, a three-dimensional accelerometer capable of detecting vibration along the axial directions of the X, Y, and Z axes is used. The detectors 33 are arranged at respective positions so that their X axes are oriented in the rotational direction of the platform 13, their Y axes are oriented in the axial direction of the platform 13, and their Z axes are oriented in the center direction of the platform 13, respectively. The detector 33 is to be arranged on at least one of the platform 13, the X-ray tube 15, the X-ray detector 16, and the cooling system 17. For example, as shown in Fig. 2 and Fig. 3, the measurement points by the detectors 33 include the positions where the X-ray tube 15 is arranged on the platform 13 and which are two positions on the front and rear sides in the axial direction of the rotation axis 14 of the platform 13, the position in the vicinity of the X-ray transmission window 21 of the X-ray tube 15, the position on the X-ray detector 16, and the position on the surface of the cooling system 17. The detector 33 is preferably arranged at all of the measurement points described above. Alternatively, the detector 33 may be arranged only at one of the points, may be arranged only at any two of the points, or may be arranged only at any three of the points. The number and the combination of the positions where the detectors 33 are arranged may be freely set as needed. As one preferable example, the detector 33 is arranged at least at the position of the X-ray tube 15 or the position where the X-ray tube 15 is arranged on the platform 13.

The data logger 34 stores chronologically the detection data detected by the detectors 33, in synchronization with the rotation of the platform 13.

The communication unit 35 wirelessly transmits the detection data detected by the detectors 33 and stored in the data logger 34, to the external device 32. In the case where the communication unit included in the X-ray CT apparatus 10 is available, the communication unit of the X-ray CT apparatus 10 may be used without using the communication unit 35.

The external device 32 includes a communication unit (external communication unit) 36, an acquisition unit 37, and a display processing unit 38. The external device 32 is configured with a computer, including a control unit which performs computation processing according to a program, a memory unit which stores a program and data, and a monitor such as a liquid crystal display. The external device 32 includes the acquisition unit 37 and the display processing unit 38 as the functions of the control unit.

The communication unit 36 is configured to wirelessly communicate with the communication unit 35 of the internal device 31, mutually.

The acquisition unit 37 acquires the detection data which are stored in the data logger 34 and which are the chronological detection data detected by the detectors 33, in synchronization with the rotation of the platform 13.

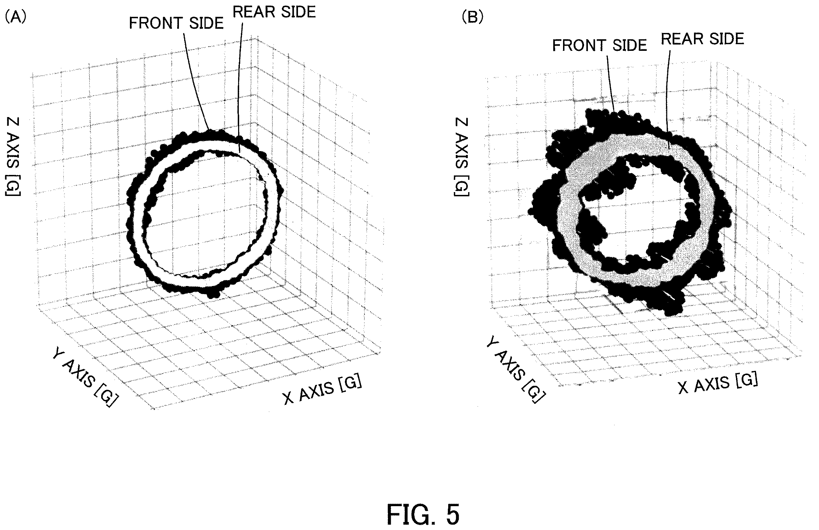

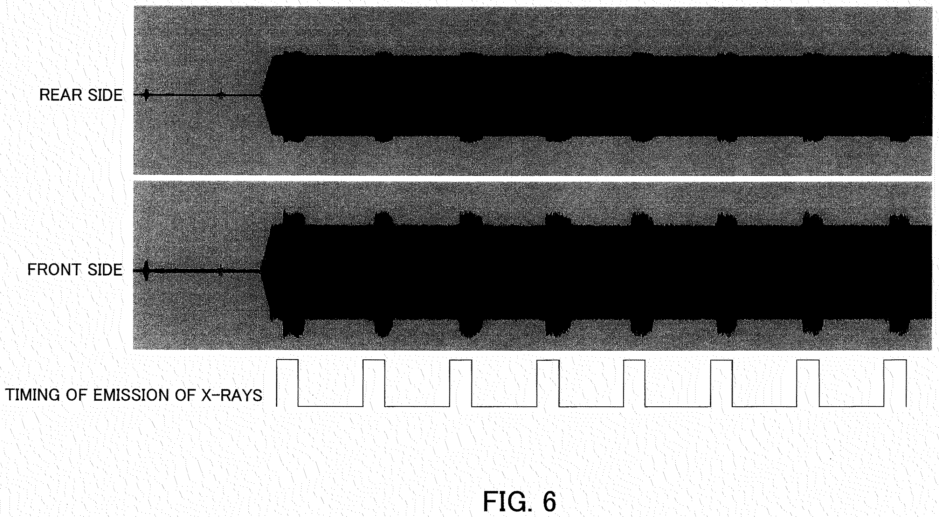

The display processing unit 38 graphically (visually) displays the chronological transition of the detection data acquired by the acquisition unit 37. The above-described displaying includes displaying changes in vibration values as a three-dimensional graphic along the respective axial directions of the X, Y, and Z axes (refer to Fig. 5), and displaying chronological changes in vibration values as a two-dimensional graphic (refer to Fig. 6).

In the case where the vibration measuring device 30 measures vibration, measurement is performed in real time with respect to the vibration in both the first state in which the platform 13 is rotated, and the X-ray tube 15, the X-ray detector 16, and the cooling system 17 have stopped operating (the first state in which the X-ray tube 15 is not emitting X-rays), and the second state in which the platform 13 is rotated, and the X-ray tube 15, the X-ray detector 16, and the cooling system 17 are operating (the second state in which the X-ray tube 15 is emitting X-rays).

The detectors 33 detect vibration along the axial directions of the X, Y, and Z axes, the data logger 34 stores chronologically the detection data detected by the detectors 33 in synchronization with the rotation of the platform 13, and the communication unit 35 transmits the detection data stored in the data logger 34 to the external device 32.

In the external device 32, via the communication unit 36, the detection data are acquired, which are stored in the data logger 34 and which are the chronological detection data detected by the detectors 33, in synchronization with the rotation of the platform 13, and thereafter the chronological transition of the acquired detection data is graphically displayed on the monitor.

Fig. 5(A) and Fig. 5(B) show examples of graphical displays of the measurement results obtained by the vibration measuring device 30, specifically, examples of three-dimensional graphical displays of the changes in vibration values along the axial directions of the X, Y, and Z axes. Fig. 5(A) graphically shows the chronological transition of the detection data synchronized with the rotation of the platform 13 in the first state in which the X-ray tube 15 is not emitting X-rays. Fig. 5(B) graphically shows the chronological transition of the detection data synchronized with the rotation of the platform 13 in the second state in which the X-ray tube 15 is emitting X-rays. Fig. 5(A) and Fig. 5(B) show the chronological transition of the changes in the vibration values along the respective axial directions of the X, Y, and Z axes, in synchronization with the rotation of the platform 13, based on the detection data detected by two detectors 33 arranged at the two positions where the X-ray tube 15 is arranged on the platform 13 and which are respectively on the front side (on the near side) and on the rear side (on the far side) in the axial direction of the rotation axis 14 of the platform 13, plotted in three-dimension as three-dimensional graphics. The respective axial directions of the X, Y, and Z axes show acceleration [G].

According to Fig. 5(A) and Fig. 5(B), the vibration values change and increase in the second state shown in Fig. 5(B) in which the X-ray tube 15 is emitting X-rays, compared to the first state shown in Fig. 5(A) in which the X-ray tube 15 is not emitting X-rays. The change in the vibration values on the front side is larger than the change in the vibration values on the rear side.

Fig. 6 is a waveform diagram of the measurement result obtained by the vibration measuring device 30. Fig. 6 shows the detection data obtained by two detectors 33 arranged at the two positions (refer to Fig. 3) where the X-ray tube 15 is arranged on the platform 13 and which are respectively on the front side (on the near side) and on the rear side (on the far side) in the axial direction of the rotation axis 14 of the platform 13, and the timing at which the X-ray tube 15 emits X-rays.

According to Fig. 6, the vibration values on both the front side and the rear side change and increase the timing at which the X-ray tube 15 emits X-rays, and the change in the vibration values on the front side is larger than the change in the vibration values on the rear side.

Similarly, with respect to the detection data detected by the detectors 33 arranged at other positions such as the position in the vicinity of the X-ray transmission window 21 of the X-ray tube 15, the position on the X-ray detector 16, and the position on the cooling system 17, their vibration values may be checked by graphically displaying the detection data, as shown in Fig. 5(A), Fig. 5(B), and Fig. 6.

When the vibration value exceeds a predetermined threshold in Fig. 5(A), Fig. (B), and Fig. 6, a warning may be displayed to indicate the fact.

The measurement of the vibration by the vibration measuring device 30 allows checking of resonant frequencies and chronological changes in resonant frequencies at the measurement points in the first state in which the X-ray tube 15 is not emitting X-rays and the second state in which the X-ray tube 15 is emitting X-rays, and matching characteristics of the X-ray CT apparatus 10 with the X-ray tube 15, the cooling system 17, and the like.

For example, when ninety-nine percent of the electric energy supplied to the X-ray tube 15 for generating X-rays is converted into heat, and the heat causes deformation of the materials such as of the anode target and the rotating body of the X-ray tube 15, and change in their rigidity, resulting in change in resonant frequency, interference with a resonant frequency of another structure may occur. This may cause resonance phenomena, thereby leading to abnormal noise, the fluctuation in tube current caused by the vibration of the cathode filament of the X-ray tube 15, damage over time, and the like.

The vibration measuring device 30 is capable of checking generation of the resonance phenomena, thus enabling feedback for design such as of the X-ray CT apparatus 10, the X-ray tube 15, and the cooling system 17. This allows for improved quality of the X-ray CT apparatus 10 and enables a reduction in a quality evaluation period of the X-ray CT apparatus 10.

Although some embodiments according to the present invention have been described, these embodiments are provided merely as examples, and are not intended to limit the scope of the invention. These novel embodiments may be embodied in other various forms, allowing various omissions, substitutions, and modifications, without departing from the scope of the invention. These embodiments and the modifications are included within the scope and the gist of the invention, and fall under the present invention described in claims below and its equivalent scope.

### Reference Signs List

- 10: X-RAY COMPUTED TOMOGRAPHY APPARATUS
- 11: CASING
- 13: PLATFORM
- 14: ROTATION AXIS
- 15: X-RAY TUBE
- 17: COOLING SYSTEM
- 30: VIBRATION MEASURING DEVICE
- 33: DETECTOR
- 34: DATA LOGGER
- 35: COMMUNICATION UNIT
- 37: ACQUISITION UNIT
- 38: DISPLAY PROCESSING UNIT

## Claims

1. A vibration measuring device of an X-ray computed tomography apparatus, the X-ray computed tomography apparatus including a casing, a platform configured to rotate in the casing, and an X-ray tube arranged on the platform, the vibration measuring device including;
a detector arranged on either one or both of the platform and the X-ray tube, the detector configured to detect vibration in a state of the platform rotating.

2. The vibration measuring device of the X-ray computed tomography apparatus according to claim 1,
the X-ray computed tomography apparatus further including a cooling system arranged on the platform and configured to cool the X-ray tube, wherein
the detector is arranged on at least one of the platform, the X-ray tube, and the cooling system.

3. The vibration measuring device of the X-ray computed tomography apparatus according to claim 1, wherein
when the detectors are to be arranged on the platform, each of the detectors is arranged at two positions on front and rear sides in an axial direction of a rotation axis of the platform.

4. The vibration measuring device of the X-ray computed tomography apparatus according to claim 3, wherein
each of the detectors is arranged at two positions where the X-ray tube is arranged on the platform, and each of the two positions is on front and rear sides in the axial direction of the platform with respect to the X-ray tube.

5. The vibration measuring device of the X-ray computed tomography apparatus according to claim 1, wherein
the detector detects vibration in a first state with the platform rotating and the X-ray tube and a cooling system having stopped operating, and in a second state with the platform rotating and the X-ray tube and the cooling system operating, respectively.

6. The vibration measuring device of the X-ray computed tomography apparatus according to claim 1, wherein
the detector is a three-dimensional accelerometer configured to detect vibration along respective directions of X, Y, and Z axes, and the X axis is oriented in a rotational direction of the platform, the Y axis is oriented in an axial direction of the platform, and the Z axis is oriented in a center direction of the platform.

7. The vibration measuring device of the X-ray computed tomography apparatus according to claim 1 including a communication unit arranged on the platform and configured to wirelessly transmit detection data detected by the detector to an external destination.

8. The vibration measuring device of the X-ray computed tomography apparatus according to claim 1 including a data logger configured to store chronologically detection data detected by the detector, in synchronization with rotation of the platform.

9. The vibration measuring device of the X-ray computed tomography apparatus according to claim 8 including an acquisition unit configured to acquire the detection data detected by the detector, in synchronization with the rotation of the platform.

10. The vibration measuring device of the X-ray computed tomography apparatus according to claim 9 including a display processing unit configured to graphically display chronological transition of the detection data acquired by the acquisition unit.

11. The vibration measuring device of the X-ray computed tomography apparatus according to claim 10, wherein
the detector is a three-dimensional accelerometer configured to detect vibration along respective directions of X, Y, and Z axes, and the X axis is oriented in a rotational direction of the platform, the Y axis is oriented in an axial direction of the platform, and the Z axis is oriented in a center direction of the platform, and
the display processing unit displays, graphically in three dimensions, changes in vibration values detected by the detector along the respective directions of the X, Y, and Z axes.

12. The vibration measuring device of the X-ray computed tomography apparatus according to claim 11, wherein
the detector detects vibration in a first state with the platform rotating and the X-ray tube and a cooling system having stopped operating, and in a second state with the platform rotating and the X-ray tube and the cooling system operating, respectively, and
the display processing unit displays, graphically in three dimensions, changes in the vibration values detected by the detector in the first state along the respective directions of the X, Y, and Z axes, and changes in the vibration values detected by the detector in the second state along the respective directions of the X, Y, and Z axes.

13. The vibration measuring device of the X-ray computed tomography apparatus according to claim 10, wherein
the display processing unit displays, chronologically and graphically in two dimensions, changes in vibration values detected by the detector.

14. The vibration measuring device of the X-ray computed tomography apparatus according to claim 13, wherein
the detector detects vibration in a first state with the platform rotating and the X-ray tube and a cooling system having stopped operating, and in a second state with the platform rotating and the X-ray tube and the cooling system operating, respectively, and
the display processing unit displays, chronologically and graphically in two dimensions, changes in the vibration values detected by the detector in the first state, and changes in the vibration values detected by the detector in the second state.

15. The vibration measuring device of the X-ray computed tomography apparatus according to claim 10, wherein
when the vibration value detected by the detector exceeds a predetermined threshold, the display processing unit displays a warning.
